# EUROPEAN PATENT APPLICATION

(11) **EP 3 939 583 A1**
(43) Date of publication of application: **19.01.2022**
(21) Application number: 21176052.5
(22) Date of filing: 28.07.2016
(51) Int. Cl.: A61K 31/353, A61K 47/44, A61K 9/00, A61K 9/02, A61P 35/00

(54) **IMPROVEMENTS IN CANCER TREATMENT WITH ISOFLAVONOIDS**

(30) Priority: 06.04.2016 US 201662318946 P
(62) Divisional of application: 16897482.2
(71) Applicant: Noxopharm Limited, Turramurra, New South Wales 2074 (AU)
(72) Inventor: KELLY, Graham, Turramurra, 2074 (AU)
(74) Representative: Carpmaels & Ransford LLP

(57) **Abstract**

The invention relates to a composition including an oleaginous base for use in a device for rectal, vaginal or urethral application and a compound of general formula (I), particularly an isoflavonoid, for improving the bioavailability of the latter, as well as its use in cancer therapy.

## Description

### Field of the invention

The invention relates to cancer therapy, especially to cytotoxic agents and chemo-sensitizing and radio-sensitising agents, particularly isoflavonoids, and to improving the bioavailability of same.

### Background of the invention

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

Plant-derived phenolic isoflavonoids have been the subject of considerable scientific research since the late-1980s. Many of these compounds have auxin or hormonal functions in plants and also display biological activities in human tissues. One of the most extensively studied plant isoflavones is genistein, remarkable for its pleiotropic actions across carcinogenesis, inflammation, cardiovascular function, and insulin resistance.

The anti-cancer activities of genistein appear to stem in part from its ability to block the phosphorylation of protein tyrosine kinases, resulting in mitotic arrest, terminal differentiation, and apoptosis of human cancer cells (Lambert et al, 2005; Williamson and Manach, 2005). Genistein also is anti-angiogenic (Piao et al, 2006). The anti-cancer effects of genistein also extend to epigenetic modifications of cancer cells through modulation of DNA methylation, miRNA-mediated regulation and histone modifications (Adjakly et al, 2015) and to inhibition of proteasome activity (Kazi et al, 2003).

Importantly, isoflavonoids have been found to be useful as cytotoxic agents, and as sensitising agents for sensitising cancer cells to cytotoxic signals from chemical or radiation insult. Some have also been shown to reverse chemo-resistance.

Despite these potentially valuable therapeutic opportunities of genistein in particular and a wide range of other related plant isoflavonoids in general, those opportunities have failed to date to be translated into the clinic. There are a number of reasons for this. One is that their biological functions are not sufficiently potent to be drug-like. Another is that there is a question as to their susceptibility to various degrees to Phase 1 and Phase 2 metabolic processes with resulting decrease in potency and bio-availability, although the extent to which these process influence therapeutic potential has not been completely understood.

Some have attempted to address these deficiencies through the synthesis of analogues of the naturally-occurring isoflavonoids, hopefully by creating new chemical entities with greater biological potency and/or being less susceptible to metabolic processes.

Idronoxil is an analogue of genistein. Idronoxil (phenoxodiol; dehydroequol; Haginin E (2H-1-Benzopyran-7-0,1,3-(4-hydroxyphenyl) is about 10x more potent as an anti-cancer agent compared to genistein, inducing cytostasis and cytotoxicity in a wide range of cancer cell types. Its biological effects include inducing apoptosis, cell cycle arrest, inhibition of angiogenesis, immune modulation and neuro-protection.

Idronoxil has proved to have better drug-like qualities compared to its parent isoflavone compound, genistein, particularly in having greater in vitro anti-cancer activity and in not being particularly susceptible to Phase 1 metabolic processes (Brown et al, 2008). However, idronoxil, in common with members of the isoflavone family, is likely susceptible to Phase 2 metabolic processes, and it is this phenomenon that is believed to account for the lack of meaningful clinical efficacy observed with this family of compounds to some extent.

Isoflavonoid molecules are highly insoluble in water. In common with other water-insoluble xenobiotics as well as water-insoluble internal hormones (steroidal hormones, thyroxine) and bile acids, the body seeks to convert these compounds into a water-soluble form that is excretable via the kidneys (Guy et al, 2008; Zhang et al, 2003). Excretion can occur via the bile, but the rate of biliary excretion is slow compared to urinary excretion, leading the body to seek to convert as much of the xenobiotic into a water-soluble form that is possible.

Compounds such as idronoxil with an underlying phenolic structure share this feature with other phenolic drugs (eg. propofol, paracetamol, naloxone).

One mode of detoxification may involve a family of UDP-glucuronyl transferase enzymes that attach the xenobiotic to the sugar, glucuronic acid, to produce a water-soluble glucuronide conjugate. A secondary, less common detoxification process involves sulfotransferase enzyme activity that yields a water-soluble sulfated conjugate.

These two families of detoxifying enzymes are located principally in the liver and the gut mucosa (King et al, 2000; Guillemette, 2003; Maruo et al, 2005; Wu et al, 2011).

Orally administered idronoxil is completely converted into water-soluble conjugates as a combined effect of transferase activity in the gut mucosa and first-pass liver metabolism; intravenously administered idronoxil also is completely conjugated, with a low level of unconjugated drug being drug retained within the cyclodextrin carrier (Howes et al, 2011).

The rate of conjugation has a significant impact on the bio-availability of isoflavonoid drugs to target tissue. Isoflavonoid glucuronyl and sulfate conjugates lack anti-cancer activity *in vitro* and require the action of glucuronidase and sulphatase enzymes to liberate the active drug candidate.

Most normal tissues generally express relatively high levels of glucuronidase and sulfatase activities, whereas tumour tissues are far more variable in their expression (Machin et al, 1980). That is, conjugated isoflavonoid drugs, as well as the broader family of phenolic drugs, are bio-available to healthy tissues because they possess the ability to deconjugate the drug, whereas their bio-availability to cancer tissue is far less certain and in some cases, non-existent.

It is even more concerning that some cancers express elevated levels of glucuronosyltransferases (Liu et al, 2015), which might explain the insensitivity of colorectal cancer cell lines (eg. HT-29, CaCo-2) to idronoxil (Alvero et al, 2008). In this setting it could be seen that the administration of isoflavonoids such as idronoxil in the oral and intravenous dosage formulations used to date, with their high levels of exposure of those isoflavonoids to Phase 2 metabolic processes, would be disadvantageous to the bio-availability of those compounds to the target tumour tissue.

Various xenobiotic detoxification systems have been observed throughout the gastro intestinal tract. The observations have been of interest and relevant to the hypothesis of a relationship between high rates of carcinogenesis in mucosa having lower detoxification potential (Peters et al. 1991). In more recent studies, the distribution and operability of detoxification systems has been observed to be highly dynamic, variable and complex (Basu et al. 2004).

Given hydrophobicity, where trialled previously, the overwhelming approach to the delivery of isoflavonoids has been to make them less hydrophobic, that is so as to improve solubility in body fluids, in an attempt to deliver them to blood and to decrease the likelihood of detoxification and excretion. Examples of these formulations include PEG and cyclodextrin conjugates.

To date a significant number of clinical trials involving isoflavonoids have been undertaken. None of these trials have demonstrated consistent efficacy. For example, Idronoxil has held an IND from the US FDA since about 2000 in both oral and intravenous dosage formulations and in that form has undergone over 12 Phase 1, Phase 2 and Phase 3 clinical studies in over 300 patients with late-stage cancers. Instances of clinical response (complete response, partial response, stable disease) have been observed, but neither dosage formulation has delivered a consistent, clinically meaningful anti-cancer effect.

The mechanism(s) underpinning that lack of consistent efficacy are not completely understood. A lack of bioavailability is clearly a concern but there is an absence of a complete understanding of the mechanisms underpinning the limited bioavailability. Confounding the point is that, quite apart from conjugation, some isoflavonoid drugs are metabolized in the liver into inactive metabolites.

To date, no isoflavonoid has obtained a marketing approval. In fact, the two last clinical studies involving isoflavonoids have failed to provide any evidence of clinical benefit. One study involving idronoxil was a Phase 3 study in patients with late-stage ovarian cancer that was abandoned following the recruitment of 142 women because of lack of any clinical benefit (Fotopoulou et al. 2014). Another study in patients with late-stage cancers and involving a compound known as ME-143 (4,4'-(7-hydroxychroman-3,4-diyl)diphenol) also failed to show any clinical benefit (Pant et al, 2014) and its clinical development was halted by its owners.

Given the amount of effort over the last 25 years that has gone into the clinical development of isoflavonoids as human therapeutics based on their potent anti-tumour effects in pre-clinical studies, it is remarkable that no isoflavonoid has come to market, perhaps pointing to a general acceptance of the research community that the clinical efficacy of isoflavonoids is limited by the inherent hydrophobicity of the molecules.

There remains a need for new cancer therapies.

There is a particular need for new formulations for inhibiting tumour growth.

There is a further need for reagents for sensitising tumour cells to the cytotoxic effects of radiotherapy and chemotherapy.

### Summary of the invention

The invention seeks to address some of the above discussed needs or limitations and in one aspect provides a composition including:
- an oleaginous base for use in a device for rectal, vaginal or urethral application;
- a compound of general formula (I) 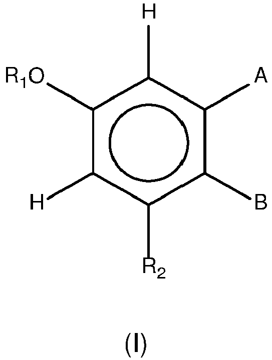 wherein
   R₁ is H, or R_{A}CO where R_{A} is C₁₋₁₀ alkyl or an amino acid;
   R₂ is H, OH, or R_{B} where R_{B} is an amino acid or COR_{A} where R_{A} is as previously defined;
   A and B together with the atoms between them form a six membered ring selected from the group 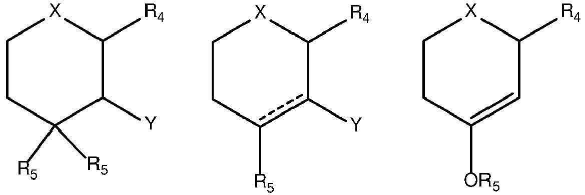 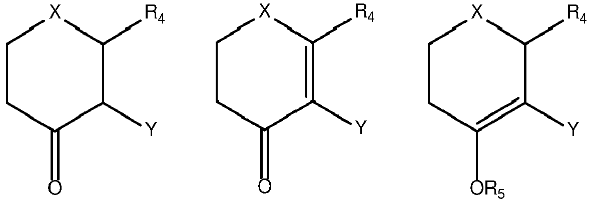 wherein
      R₄ is H, COR_{D} where R_{D} is H, OH, C₁-₁₀ alkyl or an amino acid, CO₂R_{C} where R_{C} is as previously defined, COR_{E} where R_{E} is H, C₁-₁₀ alkyl or an amino acid, COOH, COR_{C} where R_{C} is as previously defined, or CONHR_{E} where R_{E} is as previously defined;
      R₅ is H, CO₂R_{C} where R_{C} is as previously defined, or COR_{C}OR_{E} where R_{C} and R_{E} are as previously defined, and where the two R₅ groups are attached to the same group they are the same or different;
      X is O, N or S;
      Y is 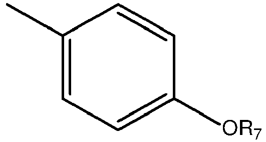
         where R₇ is H, or C₁₋₁₀ alkyl; and
         " 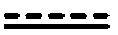 " represents either a single bond or a double bond.

Typically the compound of Formula I is:

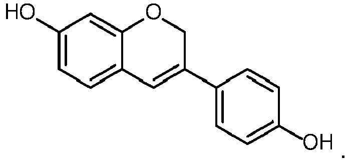

In another aspect, the invention provides a composition including:
- an oleaginous base for use in a device for rectal, vaginal or urethral application;
- a compound of general formula (II) 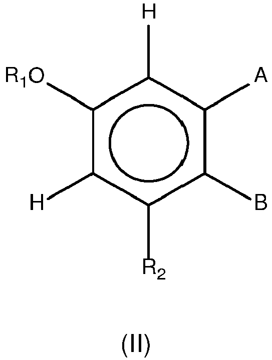 wherein
   R₁ is H, or R_{A}CO where R_{A} is C₁₋₁₀ alkyl or an amino acid;
   R₂ is H, OH, or R_{B} where R_{B} is an amino acid or COR_{A} where R_{A} is as previously defined;
   A and B together with the atoms between them form the group: 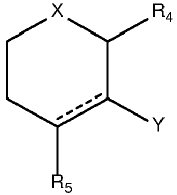 wherein
      R₄ is H, COR_{D} where R_{D} is H, OH, C₁-₁₀ alkyl or an amino acid, CO₂R_{C} where R_{C} is as previously defined, COR_{E} where R_{E} is H, C₁-₁₀ alkyl or an amino acid, COOH, COR_{C} where R_{C} is as previously defined, or CONHR_{E} where R_{E} is as previously defined;
      R₅ is substituted or unsubstituted aryl or substituted or unsubstituted heteroaryl;
      X is O, N or S;
      Y is 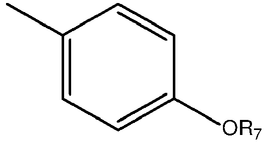
         where R₇ is H, or C₁₋₁₀ alkyl; and
         " 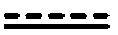 " represents either a single bond or a double bond.

Typically the oleaginous base comprises a predominance of (>45% w/w base) saturated fatty acids. Preferably the oleaginous base is Theobroma oil (cocoa butter) or an oil fraction or derivative or synthetic version thereof having a saturated fatty acid profile substantially the same as, or identical to the fatty acid profile of Theobroma oil.

In another aspect there is provided a suppository, pessary intra-urethral device or like formed from a composition described above.

In another aspect there is provided a method of treating or preventing cancer, comprising administering to a person in need thereof a suppository, pessary intra-urethral device or like described above.

In another aspect there is provided a use of a composition described above in the preparation of a suppository, pessary, intra-urethral device or like for the prevention and/or treatment of cancer.

In another aspect there is provided a suppository, pessary, intra-urethral device or like formed from a composition described above for use in preventing or treating cancer.

### Detailed description of the embodiments

Reference will now be made in detail to certain embodiments of the invention. While the invention will be described in conjunction with the embodiments, it will be understood that the intention is not to limit the invention to those embodiments. On the contrary, the invention is intended to cover all alternatives, modifications, and equivalents, which may be included within the scope of the present invention as defined by the claims.

One skilled in the art will recognize many methods and materials similar or equivalent to those described herein, which could be used in the practice of the present invention. The present invention is in no way limited to the methods and materials described.

It will be understood that the invention disclosed and defined in this specification extends to all alternative combinations of two or more of the individual features mentioned or evident from the text. All of these different combinations constitute various alternative aspects of the invention.

As used herein, except where the context requires otherwise, the term "comprise" and variations of the term, such as "comprising", "comprises" and "comprised", are not intended to exclude further additives, components, integers or steps.

As described herein, the inventor has sought to improve the clinical efficacy of isoflavonoids, especially isoflavonoids for the treatment of cancer. The inventor has investigated heretofore unexplored approaches for the clinical application of these compounds.

The inventor has recognised that it is possible to obtain a robust anti-tumour effect from isoflavonoids. As described and exemplified herein, the inventor describes the use of isoflavonoids to bring to remission an aggressive metastatic disease.

Critically, it has been found that the effect is realised when two criteria are satisfied. First, the isoflavonoid must be given in the form of a formulation having a substantially hydrophobic or lipophilic base. Second, the formulation must be given so as to enable contact of the isoflavonoid with rectal or urogenital mucosa. Where these conditions are met, the inventor has observed the anti-cancer activity of isoflavonoids against primary and metastatic disease.

Without wanting to be bound by hypothesis, an underlying mechanism of action is believed to involve the hydrophobic association of the isoflavonoid with fatty acid formulation base and the mucosal uptake of fatty acids administered in the rectal and urogenital spaces.

In more detail, when the hydrophobic base of the formulation is liquefied at body temperature in the rectal or urogenital spaces, the isoflavonoid remains hydrophobically associated (potentially by hydrophobic interactions between the fatty acid chains of the hydrophobic formulation base and the phenolic chemistry of the isoflavonoid) in the form of a fatty acid/isoflavonoid complex. In this condition, a mechanism operating at the rectal or urogenital mucosa for uptake of fatty acid chains may transport the fatty acid/isoflavonoid complex across the mucosa whereby the isoflavonoid is available for therapeutic effect.

It has been generally understood that a critical feature of suppository and pessary formulations and the like is the presence of a base associated with the pharmaceutical active that is selected to enable the partitioning of the base and active. Using the example of a suppository, where the active is hydrophilic, the suppository base is generally hydrophobic or lipophilic, enabling the active to be physically retained in the rectum until the base melts, upon which the active is released for absorption across the mucosa and the base is understood to mix with rectal fluid and to be expelled from the rectal space. In this context the base functions merely as a carrier enabling physical administration of the active.

Partitioning of active and base is very well understood to be essential to the function of a suppository. It is generally accepted that pharmaceutical actives that are highly soluble in a suppository base in fact diffuse much less rapidly out of the base than do those actives which are insoluble or have a low excipient solubility see: Allen L.V in Compounding rectal dosage forms -Part II, Secundum Artem Vol 14 No. 4 Therefore, without partitioning of active and base, when the base (hydrophilic or hydrophobic) melts or otherwise is dissolved in mucosal fluid and expelled from the rectal or urogenital space, the active is dissolved and expelled with the base. It is on the basis of this understanding that hydrophilic actives are generally formulated together with hydrophobic base (typically containing fatty acids, especially saturated fatty acids) and hydrophobic actives are generally formulated with hydrophilic base (for example cyclodextrin etc.).

Notably the invention described herein stands in contrast to these accepted principles of suppository formulation whereby the inventor has recognised that, at least insofar as certain isoflavonoids described herein are concerned, there is a surprising advantage that pertains to utilising a hydrophobic or lipophilic base, enabling the dissolution of isoflavonoids therein, and from which these isoflavonoids would be expected to diffuse less rapidly and therefore to exhibit lower partitioning. As stated, according to this invention it is believed that it is important that the active should not readily diffuse from the fatty acid base as otherwise this would mean a lesser likelihood of transfer of isoflavonoid across the rectal or urogenital mucosa on uptake of the fatty acid chains.

On the basis of the findings described herein, the inventor has recognised the applicability of isoflavonoids for treatment of cancer or sensitisation of cancer cells to chemo- or radiotherapy when given in the form of a formulation having a hydrophobic or lipophilic base .

In one aspect, therefore, the present invention provides a composition including:
- an oleaginous base for use in a device for rectal, vaginal or urethral application;
- a compound of general formula (I) or (II), as defined above.

The compounds of general formula (I) or (II) may be defined as isoflavonoids.

### A. Isoflavonoids

The isoflavonoids for use in a composition according to the invention described are shown by Formula (I) 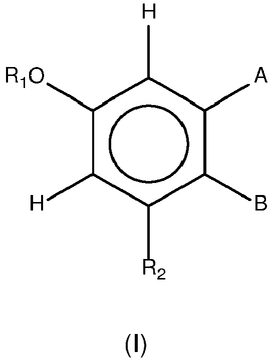 wherein
R₁ is H, or R_{A}CO where R_{A} is C₁₋₁₀ alkyl or an amino acid;
R₂ is H, OH, or R_{B} where R_{B} is an amino acid or COR_{A} where R_{A} is as previously defined;
A and B together with the atoms between them form a six membered ring selected from the group 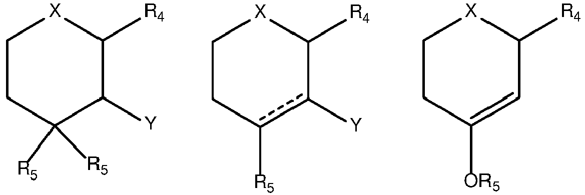 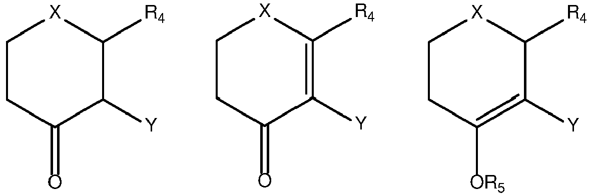 wherein
   R₄ is H, COR_{D} where R_{D} is H, OH, C₁-₁₀ alkyl or an amino acid, CO₂R_{C} where R_{C} is as previously defined, COR_{E} where R_{E} is H, C₁-₁₀ alkyl or an amino acid, COOH, COR_{C} where R_{C} is as previously defined, or CONHR_{E} where R_{E} is as previously defined;
   R₅ is H, CO₂R_{C} where R_{C} is as previously defined, or COR_{C}OR_{E} where R_{C} and R_{E} are as previously defined, and where the two R₅ groups are attached to the same group they are the same or different;
   X is O, N or S;
   Y is 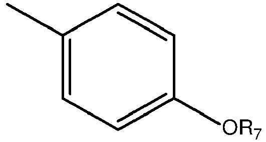
      where R₇ is H, or C₁₋₁₀ alkyl; and
      " 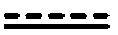 " represents either a single bond or a double bond.

Preferably, X is O.

In preferred embodiments, the compound of formula (I) is selected from the group consisting of 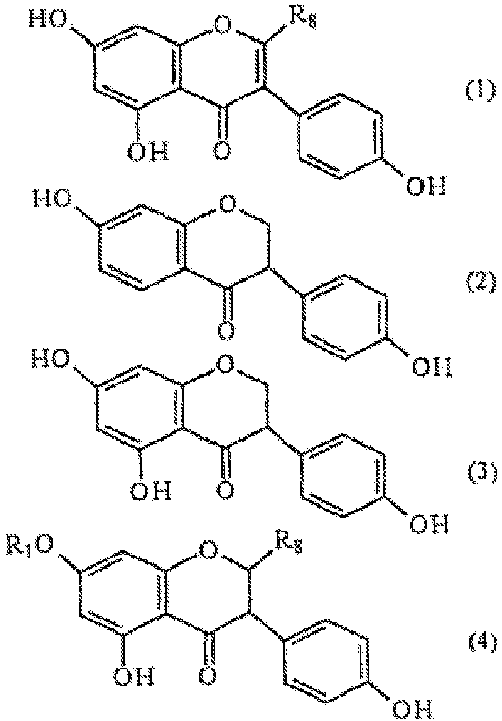 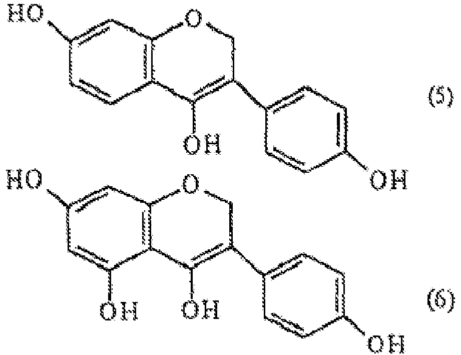 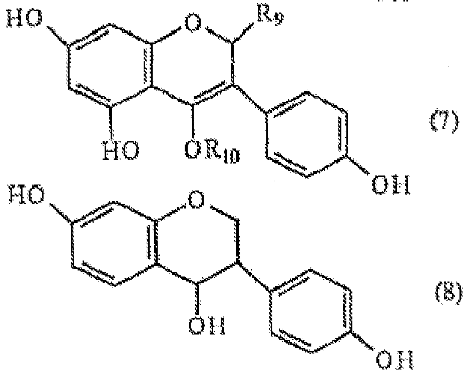 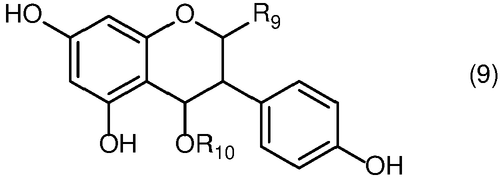 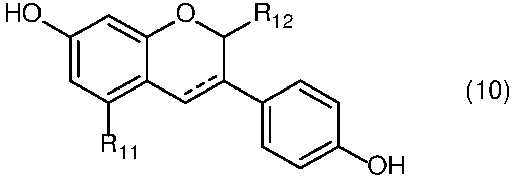 wherein
R₈ is H or COR_{D} where R_{D} is as previously defined;
R₉ CO₂R_{C} or COR_{E} where R_{C} and R_{E} are as previously defined;
R₁₀ is COR_{C} or COR_{C}OR_{E} where R_{C} and R_{E} are as previously defined;
R₁₁ is H or OH;
R₁₂ is H, COOH, CO₂R_{C} where R_{C} and is as previously defined, or CONHR_{E} where R_{E} is as previously defined; and
" 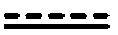 " represents either a single bond or a double bond.

Preferably, the compound of Formula (I) is 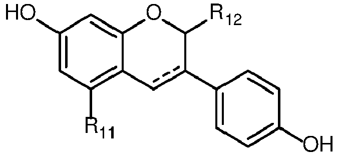 wherein R₁₁ and R₁₂ are as defined above.

Even more preferably, the compound of Formula (I) is 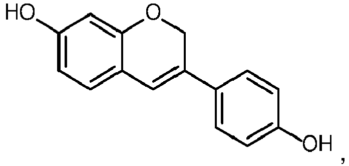
otherwise known as idronoxil (also known as phenoxodiol; dehydroequol;
Haginin E (2H-1-Benzopyran-7-0,1,3-(4-hydroxyphenyl)).

In another aspect, the isoflavonoids for use in a composition according to the invention described are shown by Formula (II): 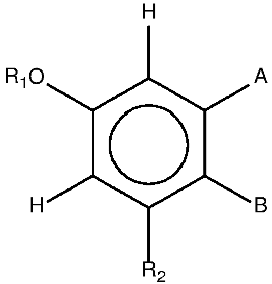 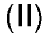 wherein
R₁ is H, or R_{A}CO where R_{A} is C₁₋₁₀ alkyl or an amino acid;
R₂ is H, OH, or R_{B} where R_{B} is an amino acid or COR_{A} where R_{A} is as previously defined;
A and B together with the atoms between them form the group: 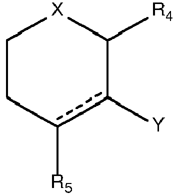 wherein
   R₄ is H, COR_{D} where R_{D} is H, OH, C₁-₁₀ alkyl or an amino acid, CO₂R_{C} where R_{C} is as previously defined, COR_{E} where R_{E} is H, C₁-₁₀ alkyl or an amino acid, COOH, COR_{C} where R_{C} is as previously defined, or CONHR_{E} where R_{E} is as previously defined;
   R₅ is substituted or unsubstituted aryl or substituted or unsubstituted heteroaryl;
   X is O, N or S;
   Y is 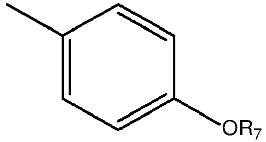
      where R₇ is H, or C₁₋₁₀ alkyl; and
      " 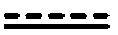 " represents either a single bond or a double bond.

In one preferred embodiment, R₅ is aryl substituted with an alkoxy group. Preferably, the alkoxy group is methoxy. In another preferred embodiment, R₅ is hydroxy.

In preferred embodiments, the compound of formula (II) is

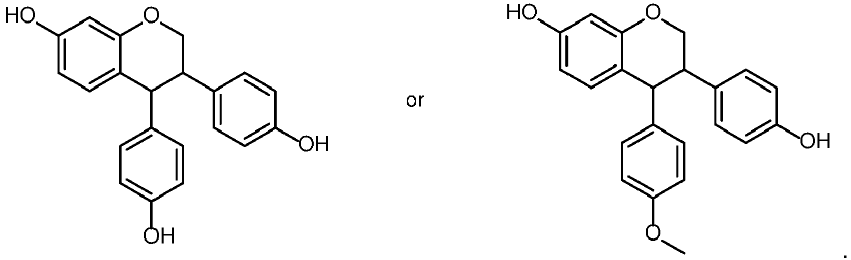

As used herein the term "alkyl" refers to a straight or branched chain hydrocarbon radical having from one to ten carbon atoms, or any range between, i.e. it contains 1, 2, 3, 4, 5, 6, 7, 8, 9 or 10 carbon atoms. The alkyl group is optionally substituted with substituents, multiple degrees of substitution being allowed. Examples of "alkyl" as used herein include, but are not limited to, methyl, ethyl, n-propyl, isopropyl, n-butyl, isobutyl, t-butyl, n-pentyl, isopentyl, and the like.

As used herein, the term "C₁₋₁₀ alkyl" refers to an alkyl group, as defined above, containing at least 1, and at most 10 carbon atoms respectively, or any range in between (e.g. alkyl groups containing 2-5 carbon atoms are also within the range of C₁₋10).

Preferably the alkyl groups contain from 1 to 5 carbons and more preferably are methyl, ethyl or propyl.

As used herein, the term "aryl" refers to an optionally substituted benzene ring. The aryl group is optionally substituted with substituents, multiple degrees of substitution being allowed.

As used herein, the term "heteroaryl" refers to a monocyclic five, six or seven membered aromatic ring containing one or more nitrogen, sulfur, and/or oxygen heteroatoms, where N-oxides and sulfur oxides and dioxides are permissible heteroatom substitutions and may be optionally substituted with up to three members. Examples of "heteroaryl" groups used herein include furanyl, thiophenyl, pyrrolyl, imidazolyl, pyrazolyl, triazolyl, tetrazolyl, thiazolyl, oxazolyl, isoxazolyl, oxadiazolyl, oxopyridyl, thiadiazolyl, isothiazolyl, pyridyl, pyridazyl, pyrazinyl, pyrimidyl and substituted versions thereof.

A "substituent" as used herein, refers to a molecular moiety that is covalently bonded to an atom within a molecule of interest. For example, a "ring substituent" may be a moiety such as a halogen, alkyl group, or other substituent described herein that is covalently bonded to an atom, preferably a carbon or nitrogen atom, that is a ring member. The term "substituted," as used herein, means that any one or more hydrogens on the designated atom is replaced with a selection from the indicated substituents, provided that the designated atom's normal valence is not exceeded, and that the substitution results in a stable compound, i.e., a compound that can be isolated, characterised and tested for biological activity.

The terms "optionally substituted" or "may be substituted" and the like, as used throughout the specification, denotes that the group may or may not be further substituted, with one or more non-hydrogen substituent groups. Suitable chemically viable substituents for a particular functional group will be apparent to those skilled in the art.

Examples of substituents include but are not limited to:
C₁-C₆ alkyl, C₁-C₆ haloalkyl, C₁-C₆ haloalkoxy, C₁-C₆ hydroxyalkyl, C₃-C₇ heterocyclyl, C₃-C₇ cycloalkyl, C₁-C₆ alkoxy, C₁-C₆ alkylsulfanyl, C₁-C₆ alkylsulfenyl, C₁-C₆ alkylsulfonyl, C₁-C₆ alkylsulfonylamino, arylsulfonoamino, alkylcarboxy, alkylcarboxyamide, oxo, hydroxy, mercapto, amino, acyl, carboxy, carbamoyl, aminosulfonyl, acyloxy, alkoxycarbonyl, nitro, cyano or halogen.

The term "isoflavonoid" as used herein is to be taken broadly and includes isoflavones, isoflavenes, isoflavans, isoflavanones, isoflavanols and similar or related compounds. Some non-limiting examples of isoflavonoid core structures are shown below: 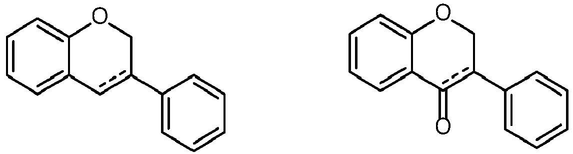 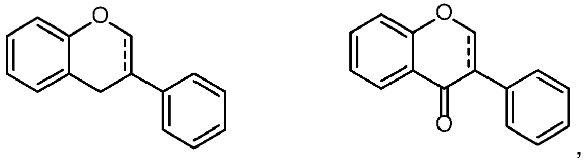 wherein " 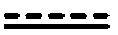 " represents either a single bond or a double bond.

Some of the compounds discussed above may be referred to by the names dihydrodaidzein (compound 1 where R₈ is H), dihydrogenestein (compounds 2 and 5), tetrahydrodaidzein (compound 8) and equol and dehydroequol (compound 10).

Methods for synthesis of the above described compounds are described in WO1998/008503 and WO2005/049008 and references cited therein towards the synthesis, the contents of which are incorporated herein by reference in entirety.

### B. Bases for forming suppository, pessary or urethral devices

As described herein, the inventor has found that oleaginous bases (i.e. hydrophobic or lipophilic bases) enable the therapeutic effect of an isoflavonoid, whereas hydrophilic bases, such as PEG, cyclodextrin and the like do not.

In the disclosure below, 'base' may refer to a substance commonly used as a carrier in a suppository, pessary or intra-urethral device.

Generally the base has a solvent power for the isoflavonoid enabling at least partial, preferably complete dissolution of the isoflavonoid in the base.

The base may be comprised of, or consist of an oil or fat.

In one embodiment the base includes saturated fatty acids in an amount of 50 to 65% w/w base. Stearic acid may be included in an amount of 25 to 40% w/w base. Palmitic acid may be included in an amount of 25 to 30% w/w base. Longer chain saturated fatty acids such as myristic, arachidic and lauric acid may be included in an amount of <2% w/w base.

Further described herein, it has been found that oleaginous bases that are high in unsaturated fatty acids tend to be less advantageous in the invention. Typically, the oleaginous base includes unsaturated fatty acids in an amount of 35 to 50% w/w base. Monounsaturated fatty acid may be included in an amount of 30 to 45% w/w base. Oleic acid may be included in an amount of 30 to 40% w/w base. Polyunsaturated fatty acids such as linoleic and alpha linolenic acid may be included in an amount of 0 to 5% w/w base.

Theobroma oil (cocoa butter) has been a traditional base in a suppository because of: (a) its non-toxic and non-irritant nature, and (b) its low melting point, meaning that it readily dissolves at body temperature when placed within a bodily cavity, However, it is increasingly being replaced for a number of reasons. One reason is its variability in composition, a consequence of its natural origins; theobroma oil also is polymorphic, meaning it has the ability to exist in more than one crystal form. Another is that the formulated product needs to be kept refrigerated because of its low melting point, rendering it unsuitable in tropical regions. This has led to a number of substitute products offering a range of advantages over theobroma oil such as greater consistency, decreased potential for rancidity, and greater ability to tailor phase transitions (melting and solidification) to specific formulation, processing, and storage requirements.

Nevertheless, theobroma oil or a fatty base with similar composition and physicochemical properties has been found to be a preferred embodiment of the invention.

Typically the oleaginous base comprises a predominance of (>45% w/w base) of saturated fatty acids. Preferably the oleaginous base is Theobroma oil (cocoa butter) or an oil fraction or derivative or synthetic version thereof having a saturated fatty acid profile substantially the same as, or identical to the fatty acid profile of Theobroma oil.

Other examples of oils that may be used to provide or obtain fatty acids useful as bases include those obtainable from natural sources such as canola oil, palm oil, soya bean oil, vegetable oil, and castor oil. Oils derived from these sources may be fractionated to obtain oil fractions containing saturated fatty acids.

The base may be formed or derived from a hard fat, butter or tallow.

A base may comprise esterified or non-esterified fatty acid chains. The fatty acid chains may be in the form of mono, di and trigycerides, preferably of saturated fatty acid chains of C9-20 chain length.

A suppository base may be formed from synthetic oils or fats, examples including Fattibase, Wecobee, Witepesoll (Dynamit Nobel, Germany), Suppocire (Gatefosse, France, Hydrokote and Dehydag.

The proportion of the oleaginous suppository base in the final product is a function of the dosage of active pharmaceutical ingredient and the presence of other pharmaceutical or inert ingredient (if any) but may be provided by way of example in an amount of about 1 to 99% w/w formulation.

### C. Manufacture

The isoflavonoid suppository, pessary and devices for urethral application of the invention may be prepared as follows. The isoflavonoid is contacted with a suppository base (as described above) in molten form in conditions enabling at least partial, preferably complete or substantially complete dissolution of the isoflavonoid in the base. This solution is then poured into a suitable mould, such as a PVC, polyethylene, or aluminium mould. For example, the isoflavonoid may be contacted with the base at a temperature of from about 35° C to about 50° C and preferably from about 40° C to about 44° C. The isoflavonoid can be milled or sieved prior to contact with the base.

It will be understood that the method for manufacture of the formulation and devices formed from same of the invention require a dissolution of the isoflavonoid in the suppository base so that the isoflavonoid is at least partially dissolved therein. In one embodiment, the conditions provided for manufacture, and formulation or device formed from same, enable at least, or provide at least, 50%, preferably 60%, preferably 70%, preferably 80%, preferably 90%, preferably 95% of the isoflavonoid for a given dosage unit to be dissolved in the dosage unit. In these embodiments, no more than 50% of the isoflavonoid for a given dosage unit, preferably no more than 40%, preferably no more than 30%, preferably no more than 20%, preferably no more than 10%, preferably no more than 5% of isoflavonoid for a given dosage unit may be in admixture with, (i.e. undissolved in) the suppository base of the dosage unit.

In a preferred embodiment, all of the isoflavonoid added to a dosage unit is dissolved in the base. In this embodiment, no isoflavonoid is left in admixture with the suppository base. This is believed to increase the likelihood of the uptake of all of the isoflavonoid given in the dosage unit.

It will be understood that the objective of the manufacture process is not to admix, or to mingle, or to blend the suppository base with the isoflavonoid as generally occurs in pharmacy practice of admixing components, as it is believed that the resulting admixture would have a lower likelihood of providing therapeutic benefit. In this context, it is particularly important that any other excipient, carrier or other pharmaceutical active does not interfere with the dissolution of the isoflavonoid in the base, for example as may occur if the isoflavonoid forms a complex with a charged molecular species (other pharmaceutical active, carrier or excipient), the result of which would be to decrease the propensity of the complex, and therefore the isoflavonoid contained in it, to dissolve in the suppository base.

Optionally the suppositories, pessaries or intra-urethral devices may be coated, prior to packing, for example with cetyl alcohol, macrogol or polyvinyl alcohol and polysorbates to increase disintegration time or lubrication or to reduce adhesion on storage.

One or more sample suppositories, pessaries, or intra-urethral devices from each batch produced are preferably tested by the dissolution method of the present invention for quality control. According to a preferred embodiment, a sample from each batch is tested to determine whether at least about 75 or 80% by weight of the base dissolves within 2 hours.

Typically the suppository, pessary or like device according to the invention is substantially hydrophobic or lipophilic throughout and does not contain a hydrophilic substance such as hydrophilic carrier or pharmaceutical active, or hydrophilic foci or region formed from the ligation or complexing of the isoflavonoid to or with another pharmaceutical compound, carrier or excipient.

Preferably the formulation for forming the suppository, pessary and devices for urethral application does not include a further pharmaceutical active, cytotoxic or chemotherapeutic agent. In this embodiment, the only active is the isoflavonoid and the formulation does not include a platin, taxane or other cytotoxic or chemotherapeutic agent.

### D. Physical characteristics

The total weight of the suppository preferably ranges from about 2250 to about 2700 mg and more preferably from about 2250 to about 2500 mg. According to one embodiment, the suppository has a total weight ranging from about 2300 mg to about 2500 mg.

The suppository or pessary is preferably smooth torpedo-shaped.

The melting point of the suppository or pessary is generally sufficient to melt in the patient's body, and is typically no more than about 37° C.

In one particularly preferred embodiment there is provided:
- a kit including:
   a plurality of suppositories sufficient in number to provide an individual with a suppository once daily, or twice daily, for a period of 30 to 90 days, preferably 30 to 60 days, preferably 30 days
   each suppository including:
      400mg or 800mg of idronoxil;
      a suppository base in the form of cocoa butter;
      wherein the suppository base in provided an amount of 1-99% w/w of the suppository,
- the kit further including :
   written instructions to provide the suppository once daily, or twice daily for a period of 30 to 90 days, preferably 30 to 60 days, preferably 30 days, preferably for use in treatment of cancer, more preferably for sensitising cancer cells to cytotoxic effect of a chemo- or radiotherapy, preferably where the cancer is prostate cancer.

### E. Methods of treatment

The formulations according to the invention in suppository, pessary, intra-urethral device or like form are useful for improving the bioavailability of isoflavonoids in a range of therapeutic applications.

In one particularly preferred embodiment, the formulations are useful for treatment of cancer, whereby the isoflavonoid is used as a cytotoxic monotherapy, or as a chemo-sensitising agent for another cytotoxic molecule.

Thus in one embodiment there is provided a method of treating or preventing cancer in an individual, including administering to a person in need thereof a suppository, pessary or intra- urethral device formed from a formulation according to the invention.

In one embodiment there is provided a use of a formulation according to the invention in the preparation of a suppository, pessary or intra- urethral device for the prevention or treatment of cancer.

In another embodiment there is provided a suppository, pessary or intra- urethral device formed from a formulation according to the invention for use in preventing or treating cancer.

Methods for applying a suppository are well known in the art. Generally the methods involve inserting the suppository to a point aligned with the inferior and medial haemorrhoid veins, thereby enabling the release of the drug to the inferior vena cave.

Methods for applying a pessary, or for urethral application of a pharmaceutically active ingredient are well known in the art.

'Treatment' generally refers to both therapeutic treatment and prophylactic or preventative measures.

Subjects requiring treatment include those already having a benign, pre-cancerous, or non-metastatic tumor as well as those in which the occurrence or recurrence of cancer is to be prevented.

The objective or outcome of treatment may be to reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder.

Efficacy of treatment can be measured by assessing the duration of survival, time to disease progression, the response rates (RR), duration of response, and/or quality of life.

In one embodiment, the method is particularly useful for delaying disease progression.

In one embodiment, the method is particularly useful for extending survival of the human, including overall survival as well as progression free survival.

In one embodiment, the method is particularly useful for providing a complete response to therapy whereby all signs of cancer in response to treatment have disappeared. This does not always mean the cancer has been cured.

In one embodiment, the method is particularly useful for providing a partial response to therapy whereby there has been a decrease in the size of one or more tumors or lesions, or in the extent of cancer in the body, in response to treatment.

"Pre -cancerous" or "pre -neoplasia" generally refers to a condition or a growth that typically precedes or develops into a cancer. A "pre -cancerous" growth may have cells that are characterized by abnormal cell cycle regulation, proliferation, or differentiation, which can be determined by markers of cell cycle.

In one embodiment, the cancer is pre-cancerous or pre -neoplastic.

In one embodiment, the cancer is a secondary cancer or metastases. The secondary cancer may be located in any organ or tissue, and particularly those organs or tissues having relatively higher hemodynamic pressures, such as lung, liver, kidney, pancreas, bowel and brain.

Other examples of cancer include blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, leukemia or lymphoid malignancies, lung cancer including small-cell lung cancer (SGLG), non-small cell lung cancer (NSGLG), adenocarcinoma of the lung and squamous carcinoma of the lung, cancer of the peritoneum, hepatocellular cancer, gastric or stomach cancer including gastrointestinal cancer, pancreatic cancer, glioblastoma, ovarian cancer, liver cancer, bladder cancer, hepatoma, breast cancer (including metastatic breast cancer), colon cancer, rectal cancer, colorectal cancer, salivary gland carcinoma, kidney or renal cancer, prostate cancer, thyroid cancer, hepatic carcinoma, anal carcinoma, penile carcinoma, testicular cancer, esophagael cancer, tumors of the biliary tract, as well as head and neck cancer.

"A condition or symptom associated" [with the cancer] may be any pathology that arises as a consequence of, preceding, or proceeding from the cancer. For example, where the cancer is a skin cancer, the condition or relevant symptom may be microbial infection. Where the cancer is a secondary tumor, the condition or symptom may relate to organ dysfunction of the relevant organ having tumor metastases. In one embodiment, the methods of treatment described herein are for the minimisation or treatment of a condition or symptom in an individual that is associated with a cancer in the individual.

In the above described embodiments, the formulation according to the invention may be useful for preventing doubling time of the cancer cells or otherwise inhibiting tumour growth, either through cytotoxic effect on the tumour cells or otherwise by generally inhibiting cell replication. In these embodiments it will be understood that the suppository formulation provides an anti neoplastic "monotherapy" effect.

In another embodiment, the method of treatment described above further includes the step of administering cytotoxic chemotherapy or radiotherapy to the individual.

In yet another embodiment there is provided a method of sensitising a cancer to chemo or radiotherapy including the steps of:
- providing an individual having a cancer in need of chemo or radiotherapy;
- administering to the individual a suppository, pessary or intra- urethral device formed from a formulation according to the invention;
- administering chemo or radio-therapy to the individual.

In another embodiment, the treatment provides for sensitisation of the tumour to radiotherapy, especially stereotactic radiotherapy. In one embodiment the treatment may provide for a reduction in tumour size utilising a sub-optimal radiation dose. It will be understood that a suboptimal radiation dose is one incapable of reducing tumour size in the absence of isoflavonoid formulation treatment.

In another embodiment, the treatment provides for sensitisation of the tumour to chemotherapy. In one embodiment, the treatment provides for a reduction in tumour size utilising a sub-optimal chemotherapy dose. It will be understood that a suboptimal chemotherapy dose is one incapable of reducing tumour size in the absence of isoflavonoid formulation treatment.

In one embodiment, the isoflavonoid formulaton treatment is provided either as a cytotoxic monotherapy, or as a radio or chemosensitising therapy according to a variable dosing regime, prior to, or at the time of radio or chemotherapy. The variable dosing regime may include an increasing dose of isoflavonoid treatment during a run in period prior to radio or chemotherapy and/or an increasing dose during radio or chemotherapy. In one example, the isoflavonoid is provided in a dose of about 400mg once daily for a period of 1 to 2 weeks and increased to 800mg once daily for a period of 1 to 2 weeks or 1 month or longer, and further increased to 1600mg (2x800mg) once daily for a period of 1 to 2 weeks or 1 month or longer. Actual amounts will be influenced by disease status, age, weight, gender and other pharmacologically relevant variables.

In one particularly preferred embodiment, the cancer is primary or secondary prostate cancer, the isoflavonoid is idronoxil and the formulation is in the form of a suppository having a suppository base formed from, or consisting of Theobroma oil (cocoa butter). The idronoxil may be contained in the suppository in an amount of 400mg or 800mg. The idronoxil may be given once or twice daily for a period of 2 to 4 weeks, or for up to 12 months.

In one embodiment, the treatment provides for an inhibition of increase in prostate specific antigen (PSA) score, or for inhibition of tumour growth. In one embodiment the treatment provides for a reduction in PSA score, preferably a 50%, 60%, 70%, 80%, 90% or 100% reduction in PSA score.

It will be understood that the formulation may also be applied in the form of a device adapted for urethral application enabling the treatment of transitional epithelial carcinoma of the bladder.

### Examples

### Example 1 Formulations

A. To make 2mL total volume suppository of 400 mg idronoxil in cocoa butter (theobroma oil): density of cocoa butter (1.72 gm per 2 mL) and density displacement factor of idronoxil (1.1). Thus, 400 mg idronoxil will displace 440 mg cocoa butter, thus need 400 mg idronoxil + 1.28 gm cocoa butter. Melt cocoa butter in water-bath at 40°C; add idronoxil; mix vigorously to obtain dissolution; spray moulds lightly with vegetable oil (eg. peanut oil); pour in cocoa butter mix; cool at 5°C; remove from mould.
B. To make 2.5 mL suppository of 500 mg equol in Fattibase, 500 mg equol is dissolved in 1.70 gm Fattibase melted to 50°C.
C. To make 2 ml suppository containing 100 mg genistein, 100 mg genistein dissolved in 1.65 gm Witsepol melted to 50°C.

### Example 2 Oral administration of isoflavonoid in theobroma oil

Patient receiving chemical castration therapy presenting with undetectable testosterone and receiving Zolodex for primary prostate cancer (PSA= 3.4). Patient received 400mg idronoxil daily by oral administration, increasing to 800mg daily for 2 weeks. PSA at completion of 2 week period = 6.7 indicating doubling time of 1 month. The oral administration failed to reduce tumour doubling time.

### Example 3 Application of isoflavonoid in high oleic acid suppository base

Patient receiving chemical castration therapy presenting with undetectable testosterone and receiving Zolodex for primary prostate cancer (PSA= 6.0). Patient received 400mg idronoxil daily in olive oil base rectally for 3 weeks. PSA at completion of 2 week period = 9.6 indicating continual growth of tumour during the treatment period.

### Example 4 Cytotoxic monotherapy with (a) 400mg and (b) 800mg phenoxodiol daily against primary prostate cancer.

Patient receiving chemical castration therapy presenting with undetectable testosterone and receiving Zolodex for primary prostate cancer has a PSA doubling time of 6 weeks (PSA= 9.6). Patient received suppository comprising 400mg idronoxil in theobroma oil suppository base daily for 4 weeks. PSA score = 10, indicating that the monotherapy with suppository formulation had effectively stopped tumour growth. Suppository treatment was stopped for a period of 2 weeks and then continued for 2 weeks. PSA score =10 indicating suppository formulation has an inhibitory effect on tumour growth.

Patient receiving chemical castration therapy having urinary obstruction and pelvic discomfort receiving 400mg idronoxil suppository formulation for primary prostate cancer (PSA= 19). Patient received suppository comprising 800mg idronoxil in theobroma oil suppository based daily in 2 week courses for 15 months. The suppository formulation slowed tumour growth to a doubling time of 6 months (PSA= 67 at completion of 15 month therapy).

### References

Alvero AB, Rossi P, Brown D, Leiser A, Kelly M, Rutherford T, Husband AJ, Mor G (2008). Phenoxodiol - a chemosensitizer in the midst of cancer chemoresistance. US Oncology. 24:39-41.
Liu H, Q L, Cheng X, et al. (2015). UDP-glucuronosyltransferase 1A determinates intracellular accumulation and anti-cancer effect of β-lapachone in human colon cancer cells. PLoS One 10:11705.
Pant S, Burns HA, Bendell JC, Kurkjian C, Jones SF, Moreno O, Kuhn JG, McMeekin S, Infante JR. (2014). A first-in-human dose-escalation study of ME-143, a second generation NADH oxidase inhibitor, in patients with advanced solid cancers. Invest New Drugs 32:87-93.
Lambert et al, 2005; Williamson and Manach, 2005
Piao M, Mori D, Satoh T, et al. (2006). Inhibition of endothelial cell proliferation, in vitro angiogenesis, and the down-regulation of cell adhesion-related genes by genistein. Combined with a cDNA microarray analysis. Endothelium 13(4):249-66
Adjakly M, Ngollo M, Dagdemir A, Judes G, Pajon A, Karsli-Ceppioglu S, Penault-Llorca F, Boiteux JP, Bignon YJ, Guy L, Bernard-Gallon D. (2015). Prostate cancer: the main risk and protective factors - epigenetic modifications. Ann Endoncrinol (Paris) 76:25-41.
Kazi A, Daniel KG, Smith DM, et al. (2003). Inhibition of the proteasome activity, a novel mechanism associated with the tumor cell apoptosis-inducing ability of genistein. Biochem Pharmacol 66(6):965-76.
Guy L, Vedrine N, Urpi-Sarda M, Gil-Izguierdo A, Al-Maharik N, Boiteaux JP, Scalbert A, Remesy C, Botting NP, Manach C. (2008). Orally administered isoflavones are present as glucosides in the human prostate. J Nutr 60:461-8.
Zhang Y, Hendrich S, Murphy PA. (2003). Glucuronides are the main isoflavone metabolites in women. J Nutr 133:399-404.
King CD, Rios GR, Green MD, Tephly TR (2000). UDP-glucuronosyltransferases. Curr Drug Metab 1:143-161.
Guillemette C. (2003). Pharmacogenomics of human UDP-glucuronosyltransferase enzymes. Pharmacogenomics J. 3:136-158.
Maruo Y, Iwai M, Mori A, Sato H, Takeuchi Y. (2005). Polymorphism of UDP-glucuronosyltransferase and drug metabolism. Curr Drug Metab 6:91-99.
Wu B, Kulkarni K, Basu S, Zhang S, Hu M. (2011). First-pass metabolism via UDP-glucuronosyltransferase: a barrier to oral bioavailability of phenolics. J Pharmaceutical Sci 100:3655-3681.
Howes JB, de Souza PL, West L, Huang LJ, Howes LG. Pharmacokinetics of phenoxodiol, a novel isoflavone, following intravenous administration to patients with advanced cancer. BMC Clin Pharmacol. 2011 Feb 3;11:1.
Machin GA, Halper JP, Knowles DM. (1980). Cytochemically demonstrable b-glucuronidase activity in normal and neoplastic lymphoid cells. Blood 56: 1111-9.
Peters WHM, Kock L, Nagengast FM, Kremers PG. (1991). Biotransformation enzymes in human intestine. Critical low levels in the colon? Gut 32:408-412.
Basu NK, Ciotti M, Hwang MS, Kole L, Mitra PS, Cho JW, Owens IS. (2004). Differential and special properties of the major human UGT1-encoded gastrointestinal UDP-glucuronosyltransferases enhance potential to control chemical uptake. J Biol Chem 279: 1429-1441.
Fotopoulou C, Vergote I, Mainwaring P, Bidzinski M, Vermorken JB, Ghamande SA, Harnett P, Del Prete SA, Green JA, Spaczynski M, Blagden S, Gore M, Ledermann J, Kaye S, Gabra H (2014). Weekly AUC2 carboplatin in acquired platinum-resistant ovarian cancer with or without oral phenoxodiol, a sensitizer of platinum cytotoxicity: the Phase III OVATURE multicentre randomized study. Ann Oncol 25:160-165.
Brown D, Heaton A, Husband A (2008). Idronoxil. Drugs Fut. 33(10):844-860.

Embodiments of the invention are set out in the following numbered clauses:
1. A composition including:
   - an oleaginous base for use in a device for rectal, vaginal or urethral application;
   - a compound of general formula (I) contained or dissolved in the base 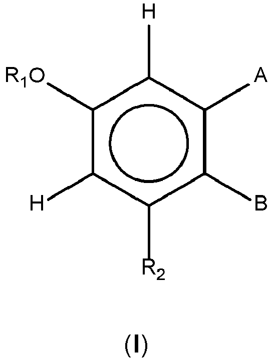 wherein
      R₁ is H, or R_{A}CO where R_{A} is C₁₋₁₀ alkyl or an amino acid;
      R₂ is H, OH, or R_{B} where R_{B} is an amino acid or COR_{A} where R_{A} is as previously defined;
      A and B together with the atoms between them form a six membered ring selected from the group consisting of: 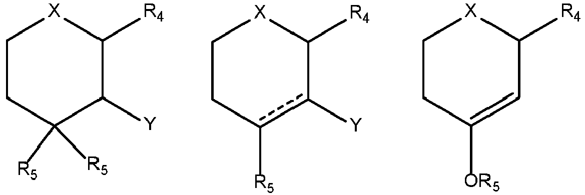 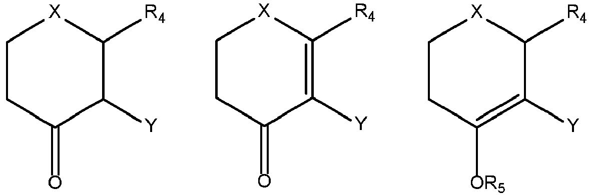 wherein
         R₄ is H, COR_{D} where R_{D} is H, OH, C₁-₁₀ alkyl or an amino acid, CO₂R_{C} where R_{C} is as previously defined, COR_{E} where R_{E} is H, C₁-₁₀ alkyl or an amino acid, COOH, CORc where Rc is as previously defined, or CONHR_{E} where R_{E} is as previously defined;
         R₅ is H, CO₂R_{C} where R_{C} is as previously defined, or COR_{C}OR_{E} where R_{C} and R_{E} are as previously defined, and where the two R₅ groups are attached to the same group they are the same or different;
         X is O, N or S;
         Y is 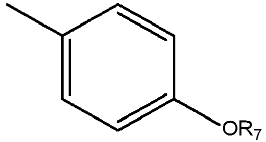
            where R₇ is H, or C₁₋₁₀ alkyl; and
            " 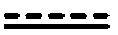 " represents either a single bond or a double bond.
2. The composition of clause 1 wherein X is O.
3. The composition of clause 1 or clause 2 wherein the compound of formula (I) is selected from the group consisting of 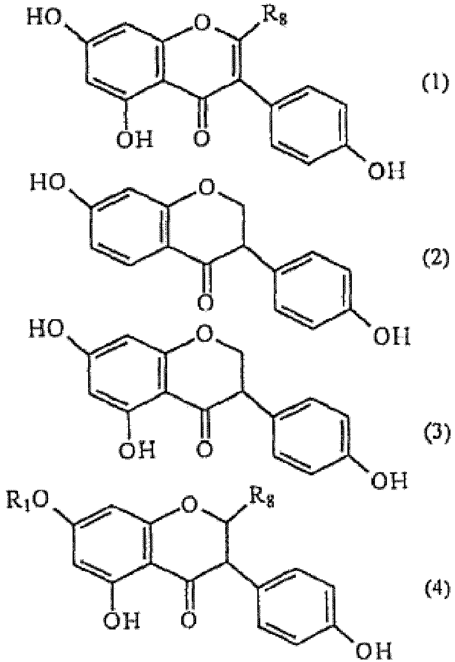 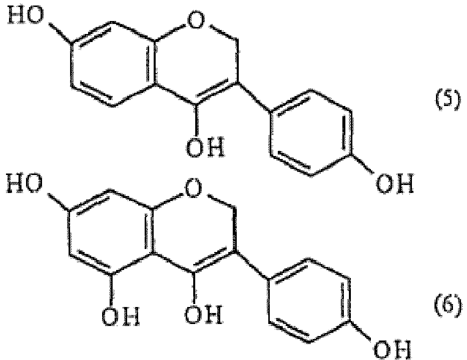 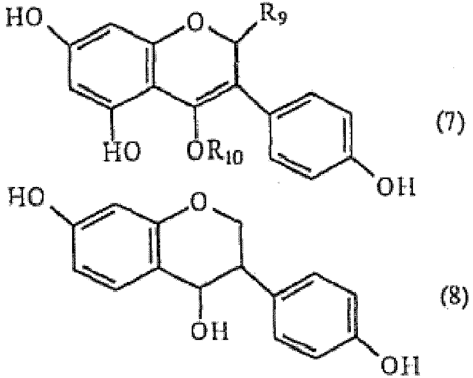 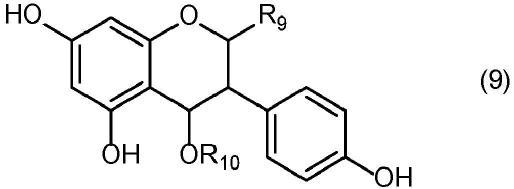 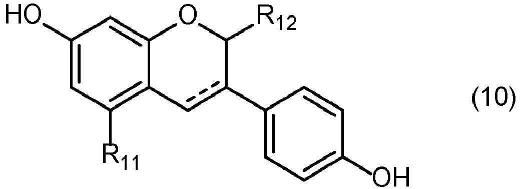 wherein
   R₈ is H or COR_{D} where R_{D} is as previously defined;
   R₉ CO₂R_{C} or COR_{E} where R_{C} and R_{E} are as previously defined;
   R₁₀ is CORc or COR_{C}OR_{E} where R_{C} and R_{E} are as previously defined;
   R₁₁ is H or OH;
   R₁₂ is H, COOH, CO₂R_{C} where R_{C} and is as previously defined, or CONHR_{E} where R_{E} is as previously defined; and
   " 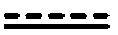 " represents either a single bond or a double bond.
4. The composition of clause 3 wherein the compound of formula (I) is 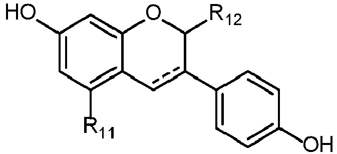 wherein R₁₁ and R₁₂ are as defined above.
5. The composition of clause 4 wherein the compound of formula (I) is 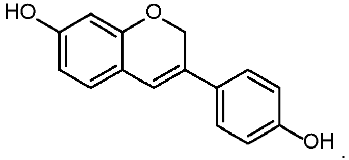
6. A composition including:
   - an oleaginous base for use in a device for rectal, vaginal or urethral application;
   - a compound of general formula (II) contained or dissolved in the base 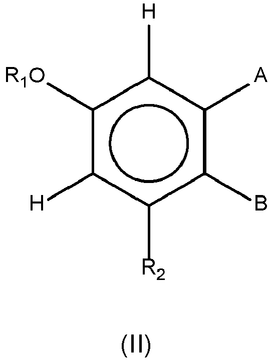 wherein
      R₁ is H, or R_{A}CO where R_{A} is C₁₋₁₀ alkyl or an amino acid;
      R₂ is H, OH, or R_{B} where R_{B} is an amino acid or COR_{A} where R_{A} is as previously defined;
      A and B together with the atoms between them form the group: 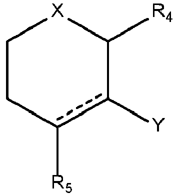 wherein
         R₄ is H, COR_{D} where R_{D} is H, OH, C₁-₁₀ alkyl or an amino acid, CO₂R_{C} where R_{C} is as previously defined, COR_{E} where R_{E} is H, C₁-₁₀ alkyl or an amino acid, COOH, CORc where Rc is as previously defined, or CONHR_{E} where R_{E} is as previously defined;
         R₅ is substituted or unsubstituted aryl or substituted or unsubstituted heteroaryl;
         X is O, N or S;
         Y is 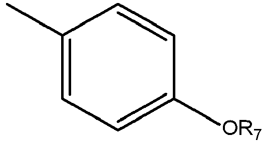
            where R₇ is H, or C₁₋₁₀ alkyl; and
            " 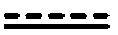 " represents either a single bond or a double bond.
7. A composition according to clause 6, wherein X is O.
8. A composition according to clause 6 or clause 7, wherein the compound of formula (II) is 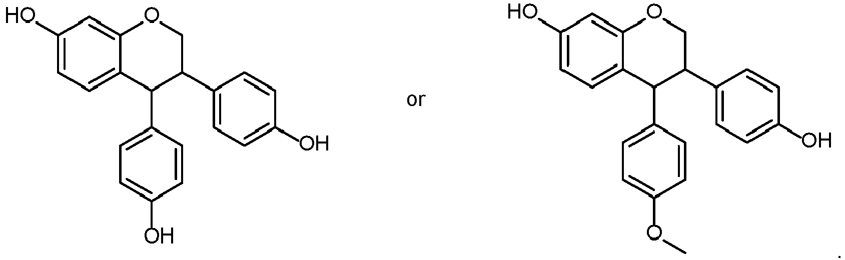
9. The composition of clause 5 or clause 8 wherein the compound is provided in the formulation in an amount of from 0.1 to 20 w/w% formulation.
10. The composition of clause 9 wherein the compound is provided in an amount of 15 w/w% formulation.
11. The composition of any one of the preceding clauses wherein the oleaginous base is provided in an amount of about 1 to 99% w/w formulation.
12. The composition of any one of the preceding clauses wherein the oleaginous base includes saturated fatty acids in an amount of 50 to 65 % w/w base.
13. The composition of clause 12 wherein the oleaginous base includes stearic acid in an amount of 25 to 40% w/w base,
14. The composition of clause 12 or 13 wherein the oleaginous base includes palmitic acid in an amount of 25 to 30% w/w base.
15. The composition of any one of clauses 12 to 14 wherein the oleaginous base includes myristic, arachidic and lauric acid in an amount of <2% w/w base.
16. The composition of any one of clauses 12 to 15 wherein the oleaginous base includes unsaturated fatty acids in an amount of 35 to 50% w/w base.
17. The composition of clause 16 wherein the oleaginous base includes monounsaturated fatty acid in an amount of 30 to 45% w/w base.
18. The composition of clause 17 wherein the oleaginous base includes oleic acid in an amount of 30 to 40% w/w base.
19. The composition of any one of clauses 16 to 18 wherein the oleaginous base includes polyunsaturated fatty acids in an amount of 0 to 5% w/w base.
20. A suppository, pessary or like formed from a composition according to any one of the preceding clauses.
21. The suppository of clause 20 wherein the suppository includes the compound 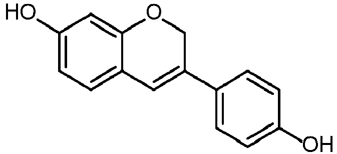 in an amount of about 40-800 mg.
22. The suppository of clause 21 wherein the oleaginous base includes Theobroma oil in an amount of about 1-99 w/w% of the suppository.
23. The suppository of clause 22 wherein the compound is dissolved in the oleaginous base.
24. A method of treating or preventing cancer, comprising administering to a person in need thereof a suppository, pessary or like according to any one of clauses 20 to 23.
25. Use of a formulation according to any one of clauses 1 to 19 in the preparation of a suppository, pessary or like for the prevention and/or treatment of cancer.
26. A suppository, pessary or like formed from a composition according to any one of the preceding clauses for use in preventing or treating cancer.

## Claims

1. A composition including:
- an oleaginous base for use in a device for rectal, vaginal or urethral application;
- a compound of general formula (I) contained or dissolved in the base 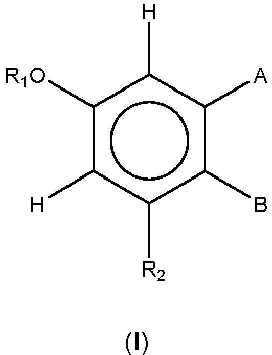 wherein
R₁ is H, or R_{A}CO where R_{A} is C₁₋₁₀ alkyl or an amino acid;
R₂ is H, OH, or R_{B} where R_{B} is an amino acid or COR_{A} where R_{A} is as previously defined;
A and B together with the atoms between them form a six membered ring selected from the group consisting of: 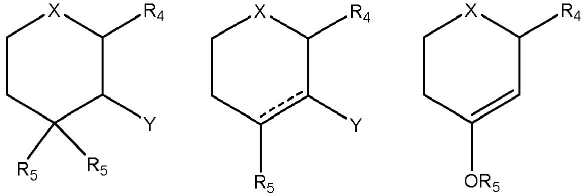 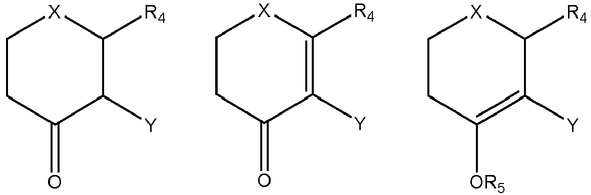 wherein
R₄ is H, COR_{D} where R_{D} is H, OH, C₁-₁₀ alkyl or an amino acid, COR_{E} where R_{E} is H, C₁-₁₀ alkyl or an amino acid, COOH,or CONHR_{E} where R_{E} is as previously defined;
R₅ is H;
X is O, N or S;
Y is 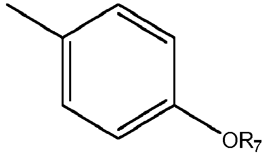
where R₇ is H, or C₁₋₁₀ alkyl; and
" 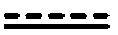 " represents either a single bond or a double bond.

2. The composition of claim 1 wherein X is O.

3. The composition of claim 1 or claim 2 wherein the compound of formula (I) is selected from the group consisting of 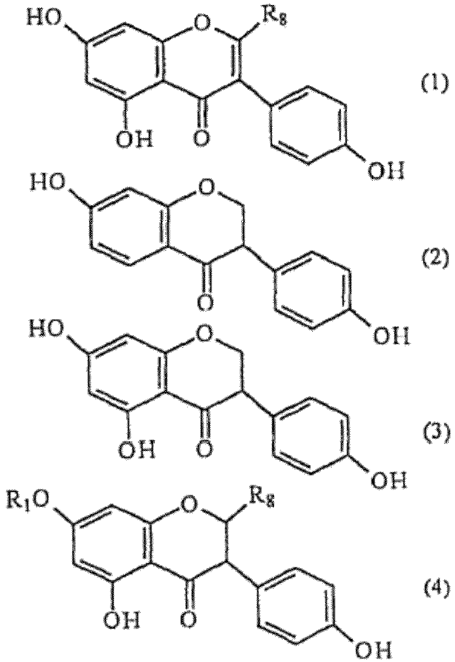 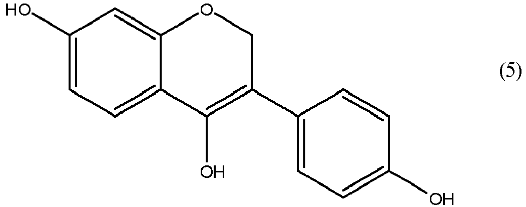 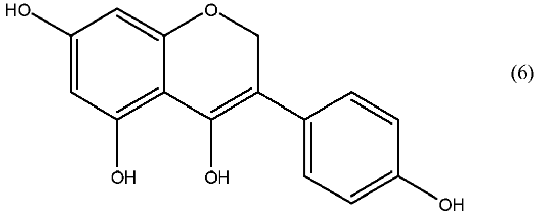 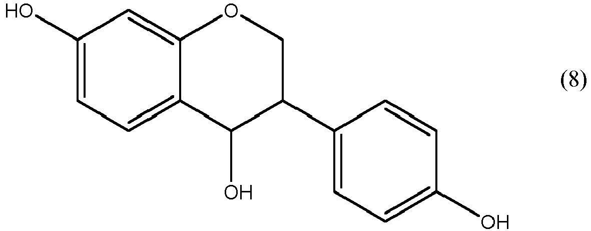 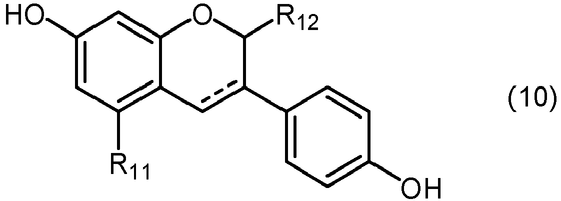 wherein
R₈ is H or COR_{D} where R_{D} is as previously defined;
R₁₁ is H or OH;
R₁₂ is H, COOH, or CONHR_{E} where R_{E} is as previously defined; and
" 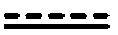 " represents either a single bond or a double bond.

4. The composition of any one of claims 1 - 3, wherein the compound of formula (I) is

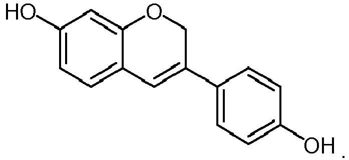

5. The composition of any one of claims 1 - 4, wherein the compound is provided in the formulation in an amount of from 0.1 to 20 w/w% formulation.

6. The composition of any one of the preceding claims wherein the oleaginous base is provided in an amount of about 1 to 99% w/w formulation.

7. The composition of any one of the preceding claims wherein the oleaginous base includes saturated fatty acids in an amount of 50 to 65 % w/w base.

8. The composition of claim 7 wherein the oleaginous base includes:
stearic acid in an amount of 25 to 40% w/w base,
and/or palmitic acid in an amount of 25 to 30% w/w base, and/or
myristic, arachidic and lauric acid in an amount of <2% w/w base.

9. A suppository, pessary or like formed from a composition according to any one of the preceding claims.

10. The suppository of claim 9 wherein the suppository includes the compound 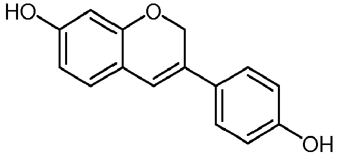 in an amount of about 40-800 mg.

11. The suppository of claim 9 or 10, wherein the compound is dissolved in the oleaginous base.

12. A suppository, pessary or like according to any one of claims 9 to 11 for use in treating or preventing cancer in a person in need thereof.
